# EUROPÄISCHE PATENTSCHRIFT

(11) **EP 3 174 468 B1**
(45) Veröffentlichungstag und Bekanntmachung des Hinweises auf die Patenterteilung: **27.06.2018**
(21) Anmeldenummer: 15744174.2
(22) Anmeldetag: 27.07.2015
(51) Int. Cl.: A61B 8/12, A61B 8/00, A61B 1/05, A61B 1/00, A61B 8/08

(54) **ENDOSKOPISCHE VORRICHTUNG**
ENDOSCOPIC DEVICE
DISPOSITIF ENDOSCOPIQUE

(30) Priorität: 28.07.2014 DE 102014214754
(43) Veröffentlichungstag der Anmeldung: 07.06.2017
(73) Patentinhaber: Digital Endoscopy GmbH, 86316 Friedberg (DE)
(72) Erfinder: PAUKER, Fritz, 86316 Friedberg (DE)
(74) Vertreter: TBK
(86) Internationale Anmeldenummer: PCT/EP2015/067155
(87) Internationale Veröffentlichungsnummer: WO 2016/016188

(56) Entgegenhaltungen:
- EP-A1- 1 803 404
- US-A- 5 265 612
- US-A- 5 762 066
- US-A1- 2002 068 870
- US-A1- 2004 210 136
- US-A1- 2012 095 292
- US-B1- 6 511 427

## Beschreibung

Die vorliegende Erfindung bezieht sich auf eine endoskopische Vorrichtung zum Einführen in einen Körperhohlraum.

Es ist ein Endoskop als ein Instrument bekannt, das in einen zu untersuchenden mitunter schwer zugänglichen und üblicherweise nicht beleuchteten Bereich, z.B. ein tunnelförmiges Lumen (Darm, Speiseröhre etc.), eingeführt wird, um sich einen Überblick über diesen zu untersuchenden Bereich zu verschaffen. Dabei wird der zu untersuchende Bereich z.B. mit Kameras abgebildet und die Abbildungsinformation dem Untersuchenden zu Verfügung gestellt. Das Dokument US-A1-2004/210316 offenbart eine endoskopische Vorrichtung gemäß dem Oberbegriff des unabhängigen Anspruchs 1.

### DURCH DIE ERFINDUNG ZU LÖSENDE AUFGABE

Die vorliegende Erfindung soll ein solches Endoskop verbessern. Es ist insbesondere die Aufgabe der vorliegenden Erfindung, eine endoskopische Vorrichtung zu schaffen, die Informationen über einen zu untersuchenden Bereich vorteilhaft zu Verfügung stellen kann.

### LÖSUNG DER AUFGABE

Diese Aufgabe ist erfindungsgemäß durch eine endoskopische Vorrichtung mit den Merkmalen von Anspruch 1 gelöst.

Vorteilhafte Weiterbildungen sind Gegenstand der abhängigen Ansprüche.

Die Erfindung betrifft somit eine endoskopische Vorrichtung zum Einführen in einen Körperhohlraum, mit einer elastographischen Aufzeichnungseinrichtung, einem Aufblaskörper, und einer Vorrichtung zum rhythmischen Verändern des Volumens des Aufblaskörpers.

Eine solche endoskopische Vorrichtung ermöglicht eine elastographische Untersuchung im Körperinneren.

Bei der Elastographie, einer relativ neuen Disziplin der Ultraschalldiagnostik, drückt der Arzt mit dem Schallkopf rhythmisch auf die zu untersuchende Stelle. Dabei werden harte Gewebeteile nicht so sehr zusammengedrückt wie weiche. Die relativen Bewegungen der Gewebeabschnitte können mittels Bildverarbeitung herausgerechnet werden. Somit können harte und weiche Gewebeabschnitte am Bildschirm unterschiedlich eingefärbt werden. In der Regel ist Krebsgewebe härter als das umliegende Gewebe und kann somit diagnostiziert werden.

Im Inneren einer Körperöffnung kann der untersuchende Arzt auf herkömmliche Weise keinen rhythmischen Druck auf das Gewebe ausüben. Bei der erfindungsgemäßen endoskopischen Vorrichtung kann der Aufblaskörper, den man ohnehin zur Ankopplung des Schalls an das Gewebe braucht, genau für diesen Zweck genutzt werden. Mit der Vorrichtung zum rhythmischen Verändern des Volumens des aufgeblasenen Aufblaskörpers kann das Volumen des Ballonkatheters rhythmisch verändert werden. Durch die abwechselnde Expansion und Kontraktion des Aufblaskörpers kann ein Druck auf das umliegende Gewebe ausgeübt werden. harte und weiche Gewebeabschnitte werden dann unterschiedlich auf den am Gewebe aufgebrachten Druck reagieren. Weiche Gewebeabschnitte werden eher und/oder stärker nachgeben als harte Gewebeabschnitte. Diese unterschiedliche Nachgiebigkeit kann elastographisch aufgezeichnet werden und bei einer eventuellen Behandlung berücksichtigt werden.

In der endoskopischen Vorrichtung kann die Vorrichtung zum rhythmischen Verändern des Volumens des Aufblaskörpers mit einer Volumenänderungsfrequenz arbeiten, die für eine elastographische Aufzeichnung geeignet ist.

Die Volumenänderungsfrequenz kann beispielsweise 1 - 2 Hz betragen, wobei auch ein Bereich zwischen 0,1 - 1 Hz vorstellbar ist. Im Prinzip kann eine beliebige Volumenänderungsfrequenz angewendet werden, die für eine vorteilhafte elastographische Aufzeichnung geeignet ist, und die unter Berücksichtigung eines Strömungswiderstandes zwischen der Vorrichtung zum rhythmischen Verändern des Volumens des Aufblaskörpers und dem Aufblaskörper technisch anwendbar ist.

In der endoskopischen Vorrichtung kann die elastographische Aufzeichnungseinrichtung ein Ultraschallkopf (Sonografie) sein. Das Prinzip der Erfindung ist auch auf andere elastographische Aufzeichnungseinrichtungen anwendbar, bei denen andere Signalarten (hörbare Schallwellen, Infraschall, Licht etc.) genutzt werden. Es ist lediglich erforderlich, dass beim zu untersuchenden Gewebe die durch die Verändern des Volumens des am Gewebe anliegenden Aufblaskörpers ermittelten harten und weichen Gewebeabschnitte durch die Signale ausreichend genau aufgezeichnet werden.

In der endoskopischen Vorrichtung kann der Aufblaskörper ein am Außenumfang der endoskopischen Vorrichtung angeordneter Ballonkatheter sein. Am distalen Ende der endoskopischen Vorrichtung könnte dann eine Kamera, eine Beleuchtungseinrichtung, ein Bildsensor oder eine andere optische Einrichtung angeordnet werden.

In der endoskopischen Vorrichtung kann alternativ der Aufblaskörper ein am distalen Ende der endoskopischen Vorrichtung angeordneter Ballonkatheter sein. In diesem Fall könnte eine Kamera, eine Beleuchtungseinrichtung, ein Bildsensor oder eine andere optische Einrichtung proximal vom Aufblaskörper angeordnet werden.

In der endoskopischen Vorrichtung kann die Vorrichtung zum rhythmischen Verändern des Volumens des Aufblaskörpers ein Wechseldruckerzeuger sein, der ein im Aufblaszustand des Aufblaskörpers im Aufblaskörper befindliches Druckmedium mit wechselndem Druck beaufschlagt. Das Druckmedium kann Wasser, Kochsalzlösung, ein wasserhaltiges Gel oder eine andere vom zu untersuchenden Körper tolerierbare Flüssigkeit sein. Das Druckmedium überträgt die Signalwellen der elastographischen Aufzeichnungseinrichtung.

In der endoskopischen Vorrichtung kann der Wechseldruckerzeuger am proximalen Ende der endoskopischen Vorrichtung angeordnet sein, und über einen in der endoskopischen Vorrichtung vorgesehenen Druckübertragungskanal mit dem Aufblaskörper verbunden ist.

In der endoskopischen Vorrichtung kann der Wechseldruckerzeuger benachbart zum Aufblaskörper angeordnet sein und mechanisch oder über eine Signalleitung, die zu einem Abschnitt der endoskopischen Vorrichtung führt, der nicht in den Körperhohlraum eingeführt wird, betätigbar sein.

In der endoskopischen Vorrichtung kann der Wechseldruckerzeuger eine Pumpe, ein Kolben oder ein zwischen zwei unterschiedlichen Drücken umschaltbarer Druckspeicher ist.

In der endoskopischen Vorrichtung kann die Vorrichtung zum rhythmischen Verändern des Volumens zwischen dem Aufblaskörper und einer Druckbeaufschlagungsvorrichtung zum Aufblasen des Aufblaskörpers angeordnet sein.

In der endoskopischen Vorrichtung können eine Beleuchtungseinrichtung und ein Bildsensor vorgesehen sein, die so angeordnet und ausgerichtet sind, dass sie den Bereich, an dem der Aufblaskörper im Körperhohlraum in Anlage gelangt, ausleuchten und abbilden können. Somit können die von der elastographischen Aufzeichnungseinrichtung ermittelten Informationen mit Bildinformation kombiniert werden, um schnell und sicher Erkenntnisse über den Zustand des fraglichen Gewebes zu erlangen.

In der endoskopischen Vorrichtung gemäß kann die endoskopische Vorrichtung ein flexibles oder starres Endoskop sein. Dadurch kann die vorliegende Erfindung flexibel bei einer Vielzahl an endoskopischen Vorrichtungen angewendet werden.

Die Merkmale der Erfindung können geeignet kombiniert werden.

Nachstehend ist die Erfindung detailliert anhand von Beispielen erläutert.

### KURZBESCHREIBUNG DER ZEICHNUNGEN

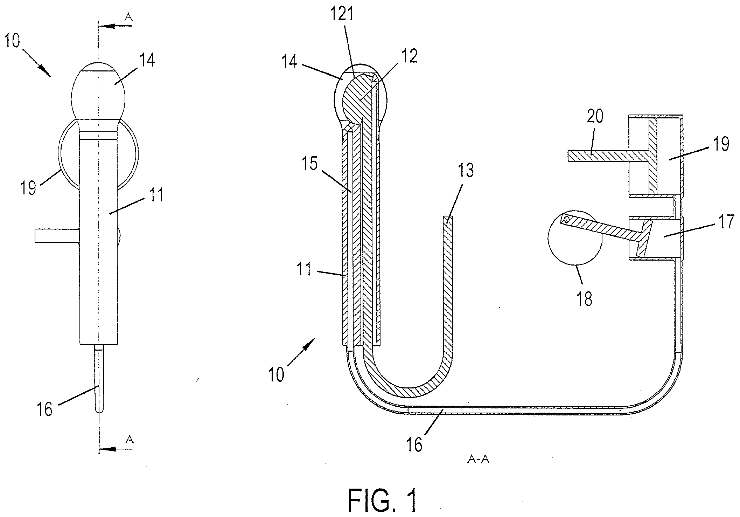

Figur 1 zeigt ein Ausführungsbeispiel einer erfindungsgemäßen endoskopischen Vorrichtung.

Nachstehend sind Ausführungsbeispiele der vorliegenden Erfindung beschrieben.

### Ausführungsbeispiel

Nachstehend ist unter Bezugnahme auf Figur 1 ein Ausführungsbeispiel erläutert.

Figur 1 zeigt ein Ausführungsbeispiel einer erfindungsgemäßen endoskopischen Vorrichtung. Insbesondere ist in Figur 1 auf der linken Seite eine Seitenansicht der endoskopischen Vorrichtung des Ausführungsbeispiels gezeigt. Darüber hinaus ist auf der rechten Seite von Figur 1 eine Schnittdarstellung entlang der Linie A-A der auf der linken Seite abgebildeten endoskopischen Vorrichtung als Draufsicht gezeigt.

Die endoskopische Vorrichtung 10 des Ausführungsbeispiels hat einen länglichen Endoskopschaft 11, der in einen zu untersuchenden Hohlraum einführbar ist. Der Endoskopschaft 11 kann starr oder flexibel sein.

Am distalen Ende des Endoskopschafts 11 ist ein Ultraschallwandler (Ultraschallkopf) 12 mit einer geeigneten Anzahl an Ultraschallzellen 121 angeordnet. Die Ultraschallzellen 121 können bei Aufbringen eines Anregungsbefehls Ultraschallwellen aussenden. Der Ultraschallwandler 12 mit den Ultraschallzellen 121 wirkt dabei als Ultraschallsonde und sendet und empfängt die Ultraschallwellen, wandelt diese in elektrische Impulse um und leitet sie zur Verarbeitung weiter. Zu diesem Zweck ist der Ultraschallwandler 12 mit einem Ultraschallwandlerkabel 13 verbunden. Im Ultraschallwandlerkabel 13 können die erlangten elektrischen Impulse, die aus den empfangenen reflektierten Ultraschallwellen generiert wurden, zu einer nicht gezeigten Verarbeitungseinheit oder Anzeigeeinheit geleitet werden. Im Ultraschallwandlerkabel 13 kann auch über eine separate Leitung der Anregungsbefehl zum Anregen der Ultraschallzellen 121 übertragen werden. Alternativ kann die Leitung für die Übertragung des Anregungsbefehls zum Anregen der Ultraschallzellen 121 eine vom Ultraschallwandlerkabel 13 separate Leitung sein.

In Figur 1 ist der Ultraschallwandler 12 in der rechten Darstellung so gezeigt, dass er die Ultraschallzellen 121 an der zum Betrachter weisenden Seite, an der nach links weisenden Seite, an der vom Betrachter weg weisenden Seite und an der nach oben weisenden Seite (die distale Seite) des Ultraschallwandlers 12 aufweist. Alternativ kann der Ultraschallwandler 12 auch so aufgebaut sein, dass er Ultraschallzellen 121 zusätzlich an der nach rechts weisenden Seite der Zeichnung besitzt. In einer weiteren Alternative kann der Ultraschallwandler 12 die Ultraschallzellen 121 auch an einer beliebigen Auswahl der vorstehend genannten Anordnungsbereiche haben.

Um den distalen Außenumfang des Ultraschallwandlers 12 herum ist ein aufblasbarer Ballon 14 angeordnet. Der aufblasbare Ballon 14 ist als Ballonkatheter ausgebildet und kann aus einem geeigneten Kunststoffmaterial bestehen und zum Beispiel transparent sein. Der aufblasbare Ballon 14 kann aus einem dehnfähigen oder nicht dehnfähigen Kunststoffmaterial bestehen, das sich bei Druckbelastung durch Aufblasen in geeignetem Maße an die zu untersuchende Wand des zu untersuchenden Hohlraums anschmiegt. Der aufblasbarer Ballon 14 ist um den Außenumfang des Ultraschallwandlers 12 herum zumindest so angeordnet, dass er zu den Ultraschallzellen 121 beabstandbar ist.

Der aufblasbarer Ballon 14 ist an seiner proximalen Seite mit einem Ballonbefüllungskanal 15 verbunden, der sich parallel zur Erstreckung des und im Inneren des Endoskopschafts 11 erstreckt. Der Ballonbefüllungskanal 15 kann ein Hohlraum im Endoskopschaft 11 sein oder als eine in den Endoskopschaft 11 fest eingeführte Röhre ausgebildet sein.

Am proximalen Ende des Ballonbefüllungskanals 15 ist der Ballonbefüllungskanal 15 mit einer Ballonbefüllungsleitung 16 verbunden, die den Ballonbefüllungskanal 15 mit einer nachstehend beschriebenen Vorrichtung zum rhythmischen Verändern des Volumens des Aufblaskörpers verbindet.

Wenn der aufblasbare Ballon 14 aufgeblasen ist, ist er zu den Ultraschallzellen 121 beabstandet.

Der aufblasbare Ballon 14 ist ausreichend beabstandet von den der Ultraschallzellen 121 am Außenumfang des Endoskopschafts 11 z.B. durch Kleben, Aufschmelzen oder ein anderes geeignetes Verfahren befestigt.

Das Material des aufblasbaren Ballons 14 kann sich alternativ von dem Bereich, an dem es am Außenumfang des Endoskopschafts 11 befestigt ist, bis zum proximalen Ende des Endoskopschafts 11 am Außenumfang des Endoskopschafts 11 erstrecken und somit eine Umhüllung des Endoskopschafts 11 bilden.

Am proximalen Ende der Ballonbefüllungsleitung 16 ist die Vorrichtung zum rhythmischen Verändern des Volumens des Aufblaskörpers angeschlossen, die im Ausführungsbeispiel als Pumpe 17 zur Impulsaufbringung ausgeführt ist. Die Pumpe 17 dient als Wechseldruckerzeuger und hat einen Zylinder, in dem ein Kolben über eine Pumpenantriebsvorrichtung 18 zu einer rhythmischen hin- und her erfolgenden Bewegung angetrieben wird. Die Pumpenantriebsvorrichtung 18 kann ein Elektromotor oder ein geeigneter anderer Antrieb sein.

Proximal (d.h. stromaufwärtig) von der Pumpe 17 ist eine Pumpe 19 zum Ballonaufblasen so angeordnet, dass sie die Ballonbefüllungsleitung 16 mit Druck beaufschlagen kann. Die Pumpe 19 hat einen Zylinder, in dem ein zylinderartiger Kolben 20 hin- und hergehend bewegbar sitzt. Der Kolben 20 kann manuell im Zylinder bewegt werden. Der Kolben 20 kann alternativ durch einen anderen geeigneten Antrieb im Zylinder bewegt werden. Der Kolben 20 wird im Zylinder der Pumpe 19 zwischen einer Druckbeaufschlagungsposition in der Zeichnung nach rechts, und einer Druckentlastungsposition in der Zeichnung nach links bewegt.

Der Druckraum der Pumpe 19 ist somit mit dem Druckraum der Pumpe 17 und der Ballonbefüllungsleitung 16 verbunden.

In dem System aus dem Druckraum der Pumpe 19, dem Druckraum der Pumpe 17, der Ballonbefüllungsleitung 16, dem Ballonbefüllungskanal 15, und dem Innenraum des Ballons 14 ist ein Druckmedium eingefüllt. Das System ist vorzugsweise abgedichtet.

Das verwendete Druckmedium ist Wasser, Kochsalzlösung, ein wasserhaltiges Gel oder eine andere vom zu untersuchenden Körper tolerierbare Flüssigkeit.

Wenn der Ballon 14 aufgeblasen ist (Druckbeaufschlagungsposition der Pumpe 19), bewirkt die hin- und her erfolgende Bewegung des Kolbens im Zylinder der Pumpe 17 eine rhythmische Veränderung des Volumens des gesamten Systems aus dem Druckraum der Pumpe 19, dem Druckraum der Pumpe 17, der Ballonbefüllungsleitung 16, dem Ballonbefüllungskanal 15, und dem Innenraum des Ballons 14. Somit bewirkt die hin- und her erfolgende Bewegung des Kolbens im Zylinder der Pumpe 17 eine rhythmische Veränderung des Volumens des Ballons 14. Somit wird im durch die Pumpe 17 beaufschlagten (gefüllten) Zustand das Volumen des Ballons 14 oszillierend mit dem Differenzvolumen, das sich zwischen der Druckbeaufschlagungsposition und der Druckentlastungsposition der Pumpe 19 ergibt, befüllt.

Die Erfindung ist nicht auf die dargestellte Pumpe 17 beschränkt. Eine beliebige andere Vorrichtung kann anstelle der Pumpe 17 angewendet werden, die zum rhythmischen Verändern des Volumens des Ballons 14 in der Lage ist.

Auch die Pumpe 19 kann einen beliebigen Aufbau haben, solange sie dazu in der Lage ist, das System aus dem Druckraum der Pumpe 19, dem Druckraum der Pumpe 17, der Ballonbefüllungsleitung 16, dem Ballonbefüllungskanal 15, und dem Innenraum des Ballons 14 mit Druck zu beaufschlagen.

### Funktion

Der Endoskopschaft 11 wird im druckentlasteten Zustand (der Kolben 20 der Pumpe 19 ist in der Zeichnung nach links ausgefahren) so weit in den zu untersuchenden Hohlraum hineingeschoben, dass der Ultraschallwandler 12 dem zu untersuchenden Bereich des Hohlraums gegenübersteht.

Der Kolben 20 der Pumpe 19 wird (in der Zeichnung nach rechts) zu der Druckbeaufschlagungsposition der Pumpe 19 eingefahren. Dadurch wird das System aus dem Druckraum der Pumpe 19, dem Druckraum der Pumpe 17, der Ballonbefüllungsleitung 16, dem Ballonbefüllungskanal 15, und dem Innenraum des Ballons 14 mit Druck beaufschlagt und der Ballon 14 aufgeblasen. Insbesondere wird der Kolben 20 so weit eingefahren, dass sich der Ballon 14 an die Hohlraumwand des zu untersuchenden Hohlraums fest anschmiegt. Die Druckbeaufschlagung erfolgt mindest derart, dass auch im Entlastungszustand der Pumpe 17 die Ballonhaut des Ballons 14 an das Gewebe des Hohlraums angedrückt bleibt.

Wenn die Ballonhaut des Ballons 14 fest an den zu untersuchenden Bereich des Hohlraums durch die Druckbeaufschlagung des Ballons 14 gedrückt ist, wird mit dem Antreiben der Pumpenantriebsvorrichtung 18 der Pumpe 17 und somit mit der rhythmischen Veränderung des Volumens im System aus dem Druckraum der Pumpe 19, dem Druckraum der Pumpe 17, der Ballonbefüllungsleitung 16, dem Ballonbefüllungskanal 15, und dem Innenraum des Ballons 14 begonnen. Dadurch wird die Ballonhaut des Ballons 14 an das Gewebe des Hohlraums rhythmisch (im gleichen Rhythmus wie die rhythmische Veränderung des Volumens im System) abwechselnd stärker und schwächer angedrückt.

Gleichzeitig mit der rhythmischen Wechselbelastung der Ballonhaut des Ballons 14 am Gewebe des Hohlraums werden die Ultraschallzellen 121 betätigt und Ultraschallaufnahmen des zu untersuchenden Bereiches des Hohlraums ausgeführt und die Ergebnisse elastgraphisch aufgezeichnet.

Die Ultraschall-gestützte Elastographie ist ein bildgebendes Verfahren, welches die Elastizität von Gewebe ermittelt und je nach Dehnungsgrad verschieden (z.B. verschieden farbig) darstellen kann. Die Ultraschall-gestützte Elastographie basiert darauf, dass sich harte Gewebeanteile - so auch Tumorgewebe oder Krebsgewebe - bei Druck weniger stark verformen als weiches Gewebe, anders ausgedrückt, diese befallenen Gewebeabschnitte verhalten sich härter als gesundes Gewebe. Aufgrund der unterschiedlichen Dehnungseigenschaften von Tumor und gesundem Gewebe entstehen minimale Zeitverschiebungen der Echosignale. Diese werden mit Hilfe einer speziellen Software auf einem Monitor der nicht gezeigten Verarbeitungseinheit oder Anzeigeeinheit in Echtzeit z.B. farbig dargestellt und auffällige Areale im Ultraschallbild markiert.

Somit können im zu untersuchenden Bereich des Hohlraums härtere und somit möglicherweise problematische Gewebeabschnitte schnell lokalisiert werden.

Danach kann die Pumpe 19 entlastet werden, wodurch sich die Ballonhaut des Ballons 14 vom Gewebe des Hohlraums trennt. Nun kann der Endoskopschaft 11 im druckentlasteten Zustand der Pumpe 19 weiter in den zu untersuchenden Hohlraum hineingeschoben werden, um einen nächsten Bereich des Hohlraums zu untersuchen.

### Vorteile

Die Untersuchung ist kurz und schmerzfrei. Die Vorrichtung ist kostengünstig.

Durch die Erfindung können lokale Verhärtungen und Gewebeveränderungen sicher, schnell und zuverlässig aufgespürt werden. Dies ermöglicht dem erfahrenen Arzt eine gezielte Entnahme von Gewebeproben. Eine Zweituntersuchung oder Zweitbiopsie kann dadurch entbehrlich werden.

Die erfindungsgemäße endoskopische Vorrichtung kann auf dem Gebiet der Gastroskopie oder Coloskopie eingesetzt werden.

### Weitere Alternativen

An der endoskopischen Vorrichtung 10 von Figur 1 kann eine Kamera, ein Bildsensor oder dergleichen am Außenumfang des Endoskopschafts 11 proximal vom aufblasbaren Ballon 14 angeordnet werden. Wenn die Ballonhaut des Ballons 14 transparent ist, kann eine Kamera, ein Bildsensor oder dergleichen auch im durch die Ballonhaut des Ballons 14 bedeckten Bereich am distalen Ende des Endoskopschafts 11 benachbart zu den Ultraschallzellen 121 am Ultraschallwandler 12 angeordnet werden.

In einer weiteren Alternative kann eine Kamera, ein Bildsensor oder dergleichen am distalen Ende des Endoskopschafts 11 und der aufblasbarer Ballon 14 proximal von Kamera, Bildsensor etc. am Außenumfang des Endoskopschafts 11 angeordnet werden.

Die Ballonhaut des Ballons 14 muss nicht transparent sein.

Im Ausführungsbeispiel sind die Pumpe 17 zur Impulsaufbringung, die Pumpenantriebsvorrichtung 18, die Pumpe 19 zum Ballonaufblasen und der Kolben 20 proximal vom Endoskopschaft 11 vorgesehen und mit dem Endoskopschaft 11 per Leitung 16 verbunden. Die Erfindung ist nicht darauf beschränkt. Die Pumpe 17 mit der Pumpenantriebsvorrichtung 18 oder die Pumpe 17 mit der Pumpenantriebsvorrichtung 18 und die Pumpe 19 und der Kolben 20 kann/können auch am proximalen Ende des Endoskopschafts 11 oder bei entsprechender Miniaturisierung am Außenumfang des Endoskopschafts 11 vorgesehen sein.

Im Ausführungsbeispiel dient die Pumpe 17 mit der Pumpenantriebsvorrichtung 18 als Wechseldruckerzeuger. Der Wechseldruckerzeuger kann auch eine gänzlich anders aufgebaute Pumpe, ein Kolben oder ein zwischen zwei unterschiedlichen Drücken umschaltbarer Druckspeicher sein. Er muss lediglich dazu in der Lage sein, rhythmisch das Volumen im aufgeblasenen und an das Gewebe angerückten Ballon 14 zu verändern.

In der endoskopischen Vorrichtung kann dieser Wechseldruckerzeuger sogar benachbart zum Ballon 14 oder im Ballon 14 angeordnet sein und mechanisch oder über eine Signalleitung, die zu einem Abschnitt der endoskopischen Vorrichtung führt, der nicht in den Körperhohlraum eingeführt wird, betätigbar sein.

Im Ausführungsbeispiel ist der Ballon 14 am Aussenumfang des Endoskopschafts 11 fest angeordnet. Der Ballon 14 muss aber nicht zwingend fest mit dem Endoskop verbunden sein. Vielmehr kann es sich auch um ein Disposable handeln, welches nach jeder Untersuchung verworfen wird. Am Endoskop befinden sich dann eine entsprechende Nut oder mehrere Nuten, in die der Ballon 14 einrasten kann. Am Ballon 14 selbst kann dann eine Verstärkung oder ein Einrastring am proximalen Ende vorgesehen sein, die/der mit der Nut oder den Nuten beim Anordnen eines neuen Ballons 14 am Endoskopschaft 11 in Einrastung gelangt.

### BEZUGSZEICHENLISTE

10 endoskopische Vorrichtung
11 Endoskopschaft
12 Ultraschallwandler
121 Ultraschallzelle
13 Ultraschallwandlerkabel
14 aufblasbarer Ballon
15 Ballonbefüllungskanal
16 Ballonbefüllungsleitung
17 Pumpe zur Impulsaufbringung
18 Pumpenantriebsvorrichtung
19 Pumpe zum Ballonaufblasen
20 Kolben

## Patentansprüche

1. Endoskopische Vorrichtung zum Einführen in einen Körperhohlraum, mit
einer elastographischen Aufzeichnungseinrichtung (12),
einem Aufblaskörper (14), und
einer Vorrichtung (17) zum rhythmischen Verändern des Volumens des Aufblaskörpers (14);
**dadurch gekennzeichnet, dass**
proximal von der Vorrichtung (17) zum rhythmischen Verändern des Volumens eine Druckbeaufschlagungsvorrichtung (19) zum Aufblasen des Aufblaskörpers (14) angeordnet ist.

2. Endoskopische Vorrichtung gemäß Anspruch 1, wobei
die Vorrichtung (17) zum rhythmischen Verändern des Volumens des Aufblaskörpers (14) mit einer Volumenänderungsfrequenz arbeitet, die für eine elastographischen Aufzeichnung geeignet ist.

3. Endoskopische Vorrichtung gemäß Anspruch 1 oder 2, wobei
die elastographische Aufzeichnungseinrichtung (12) ein Ultraschallkopf ist.

4. Endoskopische Vorrichtung gemäß einem der Ansprüche 1 bis 3, wobei
der Aufblaskörper (14) ein am Außenumfang der endoskopischen Vorrichtung angeordneter Ballonkatheter ist.

5. Endoskopische Vorrichtung gemäß einem der Ansprüche 1 bis 3, wobei
der Aufblaskörper (14) ein am distalen Ende der endoskopischen Vorrichtung angeordneter Ballonkatheter ist.

6. Endoskopische Vorrichtung gemäß einem der Ansprüche 1 bis 5, wobei
die Vorrichtung (17) zum rhythmischen Verändern des Volumens des Aufblaskörpers (14) ein Wechseldruckerzeuger ist, der ein im Aufblaszustand des Aufblaskörpers (14) im Aufblaskörper (14) befindliches Druckmedium mit wechselndem Druck beaufschlagt.

7. Endoskopische Vorrichtung gemäß Anspruch 6, wobei
das Druckmedium zum Aufblasen des Aufblaskörpers (14) Wasser, Kochsalzlösung oder eine andere vom zu untersuchenden Körper tolerierbare Flüssigkeit ist.

8. Endoskopische Vorrichtung gemäß einem der Ansprüche 1 bis 7, wobei
die Vorrichtung (17) zum rhythmischen Verändern des Volumens am proximalen Ende der endoskopischen Vorrichtung angeordnet ist, und über einen in der endoskopischen Vorrichtung vorgesehenen Druckübertragungskanal (15, 16) mit dem Aufblaskörper (14) verbunden ist.

9. Endoskopische Vorrichtung gemäß einem der Ansprüche 1 bis 7, wobei
die Vorrichtung (17) zum rhythmischen Verändern des Volumens benachbart zum Aufblaskörper (14) angeordnet ist und mechanisch oder über eine Signalleitung an einem Abschnitt der endoskopischen Vorrichtung, der nicht in den Körperhohlraum eingeführt wird, betätigbar ist.

10. Endoskopische Vorrichtung gemäß einem der Ansprüche 1 bis 9, wobei
die Vorrichtung (17) zum rhythmischen Verändern des Volumens eine Pumpe, ein Kolben oder ein zwischen zwei unterschiedlichen Drücken umschaltbarer Druckspeicher ist.

11. Endoskopische Vorrichtung gemäß einem der Ansprüche 1 bis 10, mit
einer Beleuchtungseinrichtung und einem Bildsensor, die so angeordnet und ausgerichtet sind, dass sie den Bereich, an dem der Aufblaskörper (14) im Körperhohlraum in Anlage gelangt, ausleuchten und abbilden können.

12. Endoskopische Vorrichtung gemäß einem der Ansprüche 1 bis 11, wobei
die endoskopische Vorrichtung ein flexibles oder starres Endoskop ist.

## Claims

1. Endoscopic device for insertion into a body cavity, comprising
an elastographic recording means (12),
an inflatable body (14), and
a device (17) for rhythmically changing the volume of the inflatable body (14);
**characterized in that**
a pressurizing device (19) for inflating the inflatable body (14) is arranged proximally from the device (17) for rhythmically changing the volume.

2. Endoscopic device according to Claim 1, wherein the device (17) for rhythmically changing the volume of the inflatable body (14) operates at a volume-changing frequency that is suitable for an elastographic recording.

3. Endoscopic device according to Claim 1 or 2, wherein the elastographic recording means (12) is an ultrasonic head.

4. Endoscopic device according to one of Claims 1 to 3, wherein the inflatable body (14) is a balloon catheter arranged at the outer circumference of the endoscopic device.

5. Endoscopic device according to one of Claims 1 to 3, wherein the inflatable body (14) is a balloon catheter arranged at the distal end of the endoscopic device.

6. Endoscopic device according to one of Claims 1 to 5, wherein the device (17) for rhythmically changing the volume of the inflatable body (14) is an alternating pressure generator which applies alternating pressure to a pressure medium situated in the inflatable body (14) in the inflation state of the inflatable body (14).

7. Endoscopic device according to Claim 6, wherein the pressure medium for inflating the inflatable body (14) is water, saline solution or another liquid tolerated by the body that is to be examined.

8. Endoscopic device according to one of Claims 1 to 7, wherein the device (17) for rhythmically changing the volume is arranged at the proximal end of the endoscopic device and is connected to the inflatable body (14) via a pressure transmission channel (15, 16) provided in the endoscopic device.

9. Endoscopic device according to one of Claims 1 to 7, wherein the device (17) for rhythmically changing the volume is arranged adjacent to the inflatable body (14) and can be actuated mechanically or via a signal line at a portion of the endoscopic device, which portion is not inserted into the body cavity.

10. Endoscopic device according to one of Claims 1 to 9, wherein the device (17) for rhythmically changing the volume is a pump, a piston, or a pressure accumulator switchable between two different pressures.

11. Endoscopic device according to one of Claims 1 to 10, comprising an illumination means and an image sensor, which are arranged and oriented such that they can illuminate and capture an image of the region at which the inflatable body (14) comes into abutment in the body cavity.

12. Endoscopic device according to one of Claims 1 to 11, wherein the endoscopic device is a flexible or rigid endoscope.

## Revendications

1. Dispositif endoscopique pour l'introduction dans une cavité corporelle, comprenant un dispositif d'enregistrement élastographique (12),
un corps gonflable (14), et
un dispositif (17) pour faire varier rythmiquement le volume du corps gonflable (14) ;
**caractérisé en ce que**
un dispositif de sollicitation par pression (19) est prévu en position proximale par rapport au dispositif (17) pour faire varier rythmiquement le volume, afin de gonfler le corps gonflable (14).

2. Dispositif endoscopique selon la revendication 1, dans lequel
le dispositif (17) pour faire varier rythmiquement le volume du corps gonflable (14) fonctionne avec une fréquence de variation de volume qui est appropriée pour un enregistrement élastographique.

3. Dispositif endoscopique selon la revendication 1 ou 2, dans lequel
le dispositif d'enregistrement élastographique (12) est une tête à ultrasons.

4. Dispositif endoscopique selon l'une quelconque des revendications 1 à 3, dans lequel
le corps gonflable (14) est un cathéter à ballonnet disposé au niveau de la périphérie extérieure du dispositif endoscopique.

5. Dispositif endoscopique selon l'une quelconque des revendications 1 à 3, dans lequel
le corps gonflable (14) est un cathéter à ballonnet disposé à l'extrémité distale du dispositif endoscopique.

6. Dispositif endoscopique selon l'une quelconque des revendications 1 à 5, dans lequel
le dispositif (17) pour faire varier rythmiquement le volume du corps gonflable (14) est un générateur de pression alternative qui sollicite un milieu sous pression se trouvant dans le corps gonflable (14) avec de la pression alternative dans l'état de gonflage du corps gonflable (14).

7. Dispositif endoscopique selon la revendication 6, dans lequel
le milieu sous pression pour gonfler le corps gonflable (14) est de l'eau, une solution saline ou un autre liquide tolérable par le corps à examiner.

8. Dispositif endoscopique selon l'une quelconque des revendications 1 à 7, dans lequel le dispositif (17) pour faire varier rythmiquement le volume est disposé à l'extrémité proximale du dispositif endoscopique, et est connecté au corps gonflable (14) par le biais d'une ligne de transfert de pression (15, 16) prévue dans le dispositif endoscopique.

9. Dispositif endoscopique selon l'une quelconque des revendications 1 à 7, dans lequel
le dispositif (17) pour faire varier rythmiquement le volume est disposé à côté du corps gonflable (14) et peut être actionné mécaniquement ou par le biais d'une ligne de signal au niveau d'une portion du dispositif endoscopique qui n'est pas introduite dans la cavité corporelle.

10. Dispositif endoscopique selon l'une quelconque des revendications 1 à 9, dans lequel
le dispositif (17) pour faire varier rythmiquement le volume est une pompe, un piston ou un accumulateur de pression pouvant être commuté entre deux pressions différentes.

11. Dispositif endoscopique selon l'une quelconque des revendications 1 à 10, comprenant
un dispositif d'éclairage et un capteur d'images qui sont disposés et réalisés de manière à pouvoir éclairer et reproduire la région contre laquelle le corps gonflable (14) vient s'appliquer dans la cavité corporelle.

12. Dispositif endoscopique selon l'une quelconque des revendications 1 à 11, dans lequel le dispositif endoscopique est un endoscope flexible ou rigide.
